# EUROPEAN PATENT SPECIFICATION

(11) **EP 2 023 796 B1**
(45) Date of publication and mention of the grant of the patent: **29.09.2010**
(21) Application number: 07794828.9
(22) Date of filing: 11.05.2007
(51) Int. Cl.: A61B 1/273, A61B 17/22, A61B 1/00, A61B 1/018, A61B 18/26

(54) **BALLOON CUFF**
BALLONMANSCHETTE
COUSSINET BALLON

(30) Priority: 16.05.2006 US 801675 P
(43) Date of publication of application: 18.02.2009
(73) Proprietor: Wilson-Cook Medical Inc., Winston-Salem, North Carolina 27105 (US)
(72) Inventor: SKERVEN, Gregory J., Kernersville, NC 27284 (US); KARPIEL, John A., Winston-salem, NC 27106 (US)
(74) Representative: Garratt, Peter Douglas
(86) International application number: PCT/US2007/011510
(87) International publication number: WO 2007/136599

(56) References cited:
- EP-A- 0 316 796
- WO-A-01/05311
- US-A- 5 569 161
- US-A1- 2002 193 788

## Description

This invention claims the benefit of priority of U.S. Provisional Application Serial No. 60/801,675, entitled "Balloon Cuff," filed May 16, 2006.

### TECHNICAL FIELD

The present invention relates generally to apparatus for removing stone fragments from a body passage, and in particular, to a balloon cuff configured to urge the stone fragments from the bile duct into the duodenum.

### BACKGROUND INFORMATION

It is common for various calculi, or "stones," to form within body passages, such as kidney stones in the ureter or kidneys, and gallstones in bile ducts or the gallbladder. Some stones may be harmless and may pass through the body naturally, for example, gallstones passing through the duodenum and kidney stones through the urethra. However, many other stones may become trapped and may cause serious medical problems, such as abdominal pain, fever, nausea, jaundice, and so forth. Fast and effective removal of such stones may become necessary.

In order to remove relatively large or trapped stones, it may be necessary to disintegrate a stone into smaller fragments. Several procedures are known for disintegrating the stone and subsequent removal of the smaller stone fragments.

Some common procedures for disintegrating gallstones and kidney stones include electrohydraulic lithotripsy, which uses a small probe to break up stones using shock waves generated by electricity. Similarly, laser lithotripsy may be used to break up stones by directing a controlled laser beam onto the stone surface. Another treatment option is ultrasonic lithotripsy, which uses high frequency sound waves. Alternatively, extracorporeal shock wave lithotripsy ("ESWL") may be used, which utilizes focused impulses projected from outside the body to disintegrate larger stones. Still other disintegration techniques may be used.

Once larger stones are reduced to smaller sizes using any of the above techniques, the smaller stone fragments may pass naturally through the body, or a stone removal device may be used to extract the stone fragments. Typical extraction devices comprise extraction baskets and extraction balloon catheters. An extraction basket may comprise a plurality of wires that deploy in a radially outward direction and are designed to trap the floating stones. However, drawbacks associated with extraction baskets include the need to properly orient the basket, not being able to entrap larger stone fragments in the basket, having smaller stone fragments escape between the basket wires, having the basket not being able to grasp stone fragments of awkward shapes, and having the wires of the basket get caught or snag during removal of the basket with the stones trapped therein.

As an alternative to an extraction basket, a balloon catheter may be inserted through a working lumen of an endoscope to help remove stone fragments. In an exemplary procedure, the balloon is positioned adjacent to and upstream from the stone, inflated, and then moved in a downstream direction to sweep the stone out of the bile duct and into the duodenum. The catheter may comprise multiple lumens for injection of contrast, a wire guide, and inflation of the balloon. One drawback associated with use of an extraction balloon catheter, like an extraction basket, is the need to advance the device through the working lumen of the endoscope. Due to space constraints in the endoscope, a physician typically may not perform an intraductal lithotripsy procedure, e.g., using a probe disposed through a working lumen of the endoscope, while having the extraction balloon catheter simultaneously disposed in the same or another lumen of the endoscope. Surgical time may be increased in order to sequentially perform the steps of advancing the lithotripsy probe through the working lumen, dissolving the stone, retracting the probe, inserting the balloon catheter through the working lumen, and finally attempting to catch or sweep the fragments using the balloon. Moreover, the stone fragments may have migrated due to the elapsed time and may be diffcult to relocate.

Document US 5 569 161 A discloses a system comprising an endoscope, a balloon disposed about the endoscope in a permanent manner and inflation means (see figure 9 of said document).

Accordingly, there is a need for an improved stone extraction device that is easy to use and reduces the operation time during a stone removal procedure. There is also a need for a retrieval device that may be disposed in the vicinity of the stone fragments while an intraductal lithotripsy procedure is simultaneously performed through an endoscope.

### SUMMARY

The present invention is set forth in the appended claims. There is provided a balloon cuff comprising an attachment structure adapted to be disposed about an exterior surface of an endoscope, and a balloon coupled to the attachment structure. An inflation means is in fluid communication with the balloon. The balloon has a deflated state suitable for insertion into a body passage and an inflated state suitable for urging stone fragments along the body passage. For example, if gallstone fragments are trapped in the bile duct, the balloon may urge the stone fragments into the duodenum so that the stone fragments may pass out of the body naturally.

In a first embodiment, the attachment structure comprises a cuff member adapted to be secured about the exterior surface of the first endoscope, preferably near the distal end of the first endoscope. The cuff member may be elastic so that it is secured about the exterior surface of the first endoscope. Optionally, a securing means, such as an adhesive, tie-down band, cable-tie, heat-shrink tubing and the like may be used to secure the cuff member to the exterior surface of the first endoscope. In an alternative embodiment, the cuff member may be longitudinally movable about the exterior surface of the first endoscope. Advantageously, in either embodiment, any conventional endoscope may be retrofitted to employ such a balloon cuff.

In another embodiment, a system is provided and comprises a first endoscope, a balloon cuff secured about the exterior surface of the first endoscope, and a probe disposed through the working channel of the first endoscope. In an exemplary procedure, an intraductal shock wave lithotripsy procedure may be performed using the probe inserted through the working lumen of the first endoscope. Stone fragments may then be removed using the balloon cuff secured to the exterior surface of the first endoscope. Advantageously, since the balloon cuff is disposed on the exterior surface of the first endoscope as the probe disintegrates the stone, the probe need not be removed from the working lumen for insertion of a separate stone retrieval device.

In a first method of operation, a stone may be removed percutaneously. For example, in the case of a gallstone, a wire guide may be advanced through the cystic duct and into the bile duct. The first endoscope then is advanced over the wire guide with the balloon cuff disposed thereon, the balloon being in the deflated state. The first endoscope is disposed proximal (upstream) to the gallstone and the balloon may be inflated to occlude the bile duct. In a next step, a shock wave lithotripsy probe may be advanced through the working lumen of the first endoscope to disintegrate the gallstone. The first endoscope, having the inflated balloon disposed thereon, then is advanced distally (downstream) to urge stone fragments into the duodenum so they may pass naturally.

In an alternative method, the gallstone may be removed endoscopically using the first endoscope in conjunction with a larger, second endoscope, e.g., a duodenoscope. The second endoscope has proximal and distal ends and a working lumen sized to receive the first endoscope having the balloon cuff disposed thereon. In use, the second endoscope may be disposed adjacent the sphincter of Oddi, while the first endoscope having the balloon cuff disposed thereon is advanced into the bile duct. If necessary, intraductal shock wave lithotripsy may be performed to disintegrate the gallstone. The first endoscope having the balloon cuff then is advanced distal (upstream) to the stone fragments, the balloon is inflated, and the device is retracted proximally (downstream) to urge stone fragments into the duodenum.

Other systems, features and advantages of the invention will be, or will become, apparent to one with skill in the art upon examination of the following figures and detailed description. It is intended that all such additional systems, features and advantages be within the scope of the invention, and be encompassed by the hollowing claims.

### BRIEF DESCRIPTION OF THE DRAWINGS

The invention can be better understood with reference to the following drawings and description. The components in the figures are not necessarily to scale, emphasis instead being placed upon illustrating the principles of the invention. Moreover, in the figures, like referenced numerals designate corresponding parts throughout the different views.

FIG. 1 is a perspective view of a balloon cuff in accordance with a first embodiment.

FIG. 2 is a perspective view showing the balloon cuff of FIG. 1 disposed over an exterior surface of a first endoscope.

FIG. 3 is a perspective view illustrating securing means that may be used to secure the cuff member and the inflation means of FIG. 1-2 to the exterior surface of the first endoscope.

FIG. 4 is a perspective view of an alternative cuff member that may move longitudinally with respect to an endoscope.

FIGS. 5A-5E describe a first method for removing a stone or multiple stone fragments from a body passage.

FIGS. 6A-6F describe an alternative method for removing a stone or multiple stone fragments from a body passage.

FIG. 7 illustrates an alternative use of a balloon cuff.

FIG. 8 is an end view of an alternative embodiment comprising a balloon adapted to be disposed over an exterior surface of an endoscope.

FIG. 9 illustrates an end view of an alternative embodiment of FIG. 8.

FIG. 10 illustrates the balloon of FIG. 8 disposed over an exterior surface of an endoscope.

### DETAILED DESCRIPTION OF THE PREFERRED EMBODIMENTS

In the present application, the term "proximal" refers to a direction that is generally towards a physician during a medical procedure, while the term "distal" refers to a direction that is generally towards a target site within a patent's anatomy during a medical procedure. Thus, "proximal" and "distal" directions relative to the bodily passageway in which the procedure is being performed, depend on the point of entry for the procedure (e.g., percutaneously or endoscopically).

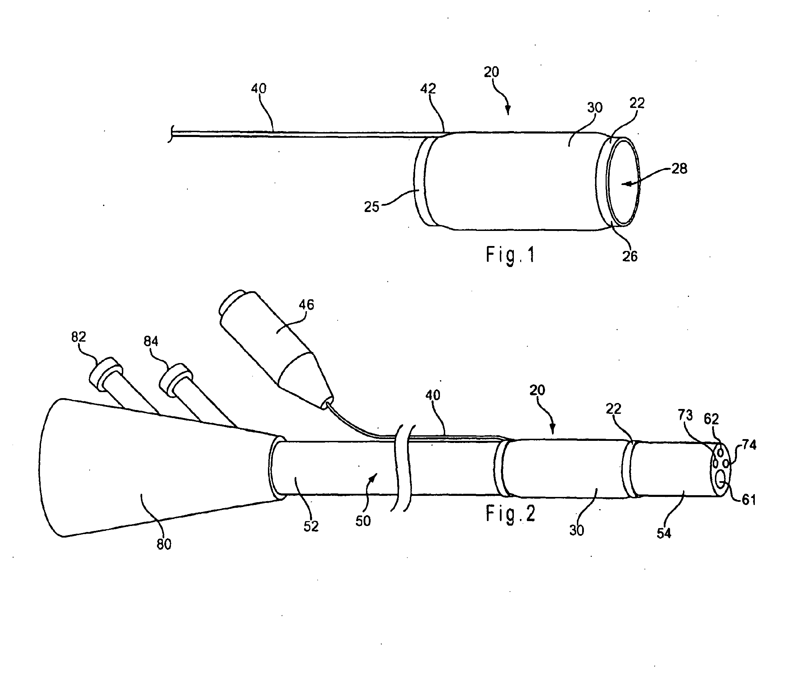

Referring now to FIG. 1, a first embodiment of a balloon cuff 20 is described. The attachment structure preferably comprises cuff member 22 having proximal end 25, distal end 26, and lumen 28 disposed therebetween. Balloon cuff 20 further comprises balloon 30, which preferably is disposed on an exterior surface of cuff member 22, as shown in FIG. 1.

The apparatus further comprises inflation means 40, which preferably comprises a tube having proximal and distal ends. Distal end 42 of inflation means 40 is in fluid communication with balloon 30 and includes an inflation lumen for the passage of an inflation fluid that may be used to selectively inflate and deflate the balloon.

Balloon 30 may comprise any balloon material and configuration that is known in the art, e.g., it may comprise an elastic material of the type typically used for extraction balloons. Without limitation, examples of such materials may include silicone and latex. However, balloon 30 may alternatively comprise an inelastic material of the type typically used for performing balloon angioplasty, deploying a stent, or other interventional purposes. Examples of such inelastic materials may include nylon, PET, and Pebax. Finally, balloons having an intermediate compliancy, for example, made of various types of polyurethane materials may be used, or alternatively, balloon 30 may be formed from hybrid or coextruded materials.

As will be explained in further detail below, balloon 30 is configured to urge stone fragments in a direction along a body passage when in the inflated state and engaged with a wall of the body passage. To achieve this function, balloon 30 may comprise various features. For example, balloon 30 may comprise a material having a softness and elasticity that allows the balloon to move with respect to the body lumen wall while the expanded balloon maintains contact with the lumen wall. Therefore, balloon 30 may fill the body lumen and engage the lumen wall in the expanded state, but is not prevented from movement to sweep stone fragments along or out of the passage.

In addition to the provision of a material having a desired softness and elasticity, or as an alternative, balloon 30 may comprise a lubricious coating to facilitate movement with respect to a body lumen wall while the expanded balloon maintains contact with the lumen wall. Also, the outer surface of balloon 30 may comprise a material having a low coefficient of friction to thereby facilitate a sweeping movement of the balloon inside the body passage. Still further, balloon 30 may be inflated to a diameter less than its maximum potential diameter, so that in the underinflated diameter, the balloon engages the lumen wall in the inflated state, but does not exert a pressure against the wall that inhibits a sweeping movement of the balloon inside the body passage. Regardless of the technique used, it is important that balloon 30 is not prevented from moving with respect to the body passage wall when in an inflated state.

In one embodiment, distal end 42 on inflation means 40 is adhered to proximal end 25 of cuff member 22 to securely couple the inflation means to the balloon cuff. A small aperture (not shown) may be disposed on a proximal region of balloon 30. Distal end 42 of inflation means 40 may extend into the aperture of the balloon to permit fluid communication therebetween. The aperture then may be sealed around the tubing to prohibit leakage.

The proximal end of inflation means 40 is coupled to actuation device 46, as depicted in FIG. 2. In one embodiment, actuation device 46 may comprise a button (not shown) configured to enable the physician to selectively inflate and deflate balloon 30. The proximal end of actuation device 46 may be coupled to an inflation source (not shown) capable of providing fluid such as air, saline, and the like to inflation means 40 via actuation device 46. In another embodiment, actuation device 46 may be a self-contained syringe that may be actuated to selectively inflate and deflate balloon 30.

In one embodiment, cuff member 22 comprises an elastic member, such that lumen 28 has a first inner diameter in a relaxed state, but when expanded radially outward, lumen 28 may assume a second, slightly larger inner diameter. Lumen 28 is sized to be disposed on an exterior surface of a first endoscope, such as cholangioscope 50 of FIG. 2. If cuff member 22 is elastic, it may be sized such that its inner diameter in the relaxed state is slightly smaller than the exterior diameter of cholangioscope 50, but its inner diameter in the expanded state is slightly larger than an exterior diameter of cholangioscope 50. Therefore, cuff member 22 may be elastically expanded to fit over distal end 54 of cholangioscope 50, then advanced proximally. Once in place, in the relaxed state cuff member 22 will be securely engaged around the exterior surface of cholangioscope 50 using a frictional fit, as shown in FIG. 2. The interior surface of cuff member 22 may comprise a texture or material, such as rubber, to increase the frictional contact with the exterior surface of cholangioscope 50.

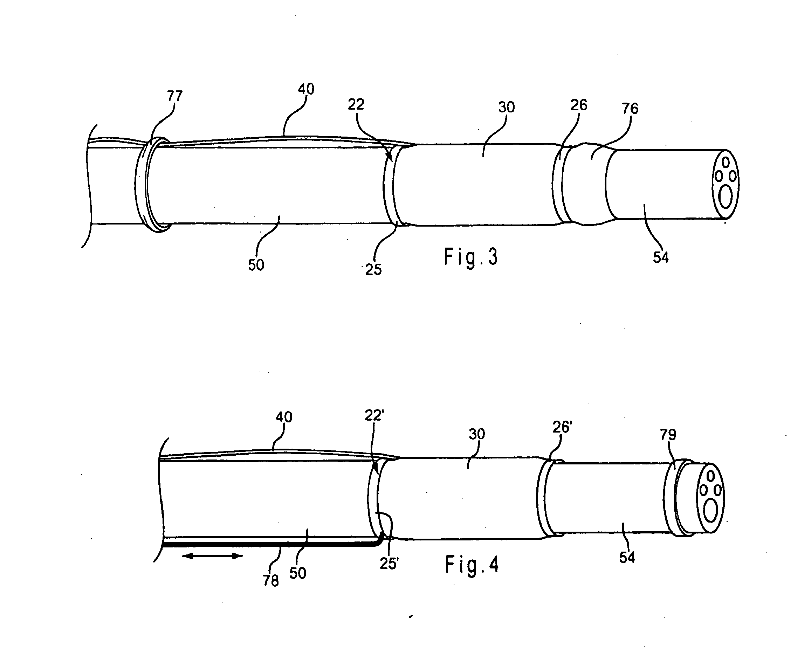

If desired, a securing means may be applied to secure proximal end 25 and/or distal end 26 of cuff member 22 directly to an exterior surface of cholangioscope 50 to inhibit movement of cuff member 22 with respect to cholangioscope 50. For example, in FIG. 3, securing means 76 is employed to secure distal end 26 of cuff member 22 to cholangioscope 50. Securing means 76 may comprise an adhesive tape, heat-shrink tubing, one or more tie-down bands, cable-ties, and the like. Securing means 76 is configured and disposed so as to not interfere with inflation of balloon 30 and movement of cholangioscope 50 through the patient.

Inflation means 40 also may be secured to the exterior surface of cholangioscope 50 using one or more securing means 77. Inflation means 40 may be secured to cholangioscope 50 in a manner similar to cuff member 22, e.g., using an adhesive tape, heat-shrink tubing, one or more tie-down bands, and the like.

In an alternative embodiment, shown in FIG. 4, cuff member 22' may be moveable when disposed on cholangioscope 50. In this embodiment, cuff member 22' preferably has a more rigid configuration, e.g., manufactured from rigid plastic or stainless steel. Cuff member 22' preferably has a non-variable inner diameter that is slightly larger than an external diameter of cholangioscope 50 and is disposed for longitudinal movement with respect to cholangioscope 50, for purposes explained below. In this embodiment, push rod 78 may be coupled to proximal end 25 of cuff member 22' to help distally advance cuff member 22' towards distal end 54 of cholangioscope 50. Optionally, distal stop means 79, which is secured around the distal end of cholangioscope 50, may be employed to ensure that cuff member 22' may not be advanced distally over the end of cholangioscope 50.

Referring back to FIG. 2, exemplary cholangioscope 50 may be any conventional endoscope known in the art. For example, cholangioscope 50 may comprise optical elements 73 and 74, which employ fiber optic components for illuminating and capturing an image distale to the cholangioscope. Further, cholangioscope 50 preferably comprises working lumen 61 and auxiliary lumen 62, which are in communication with proximal ports 82 and 84 of hub module 80, respectively.

Proximal port 84 and auxiliary lumen 62 preferably are sized to accommodate the insertion of a wire guide therethrough. Additionally, auxiliary lumen 62 may be configured to provide a fluid, such as an electrohydraulic fluid used during an electrohydraulic lithotripsy procedure, or alternatively, an irrigating fluid such as saline.

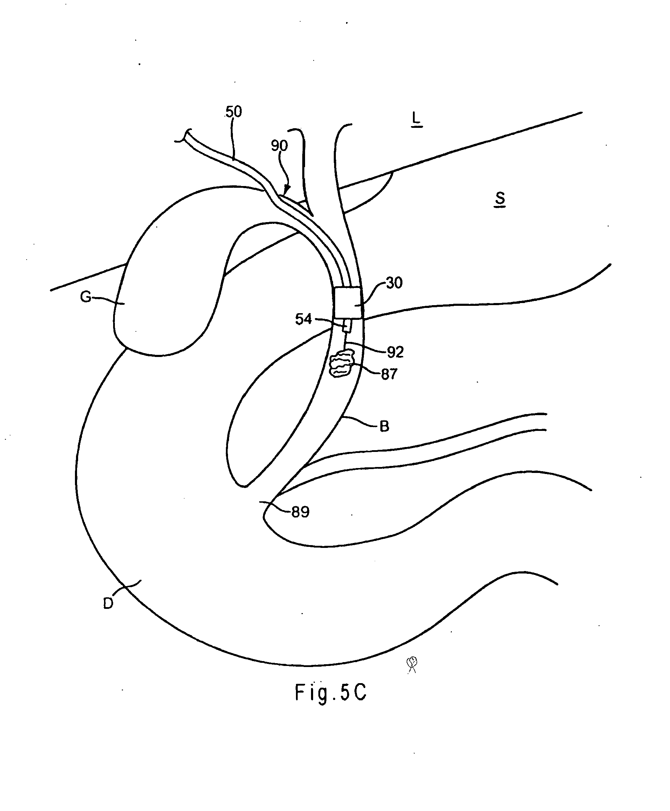

Proximal port 82 and working lumen 61 preferably are sized to accommodate a shock wave lithotripsy device, as will be explained in more detail below. In one embodiment, the lithotripsy device is in the form of shock wave probe 92 (see FIG. 5C), which may generate shock waves at the surface of a stone to help break-up or dissolve the stone. If electrohydraulic lithotripsy is performed, as described below, an electrohydraulic generator unit (not shown) may be coupled to shock wave probe 92 to generate the required energy.

It will be apparent to one skilled in the art that while one auxiliary lumen 62 and one working lumen 61 are shown, cholangioscope 50 may comprises any number of lumens/channels.

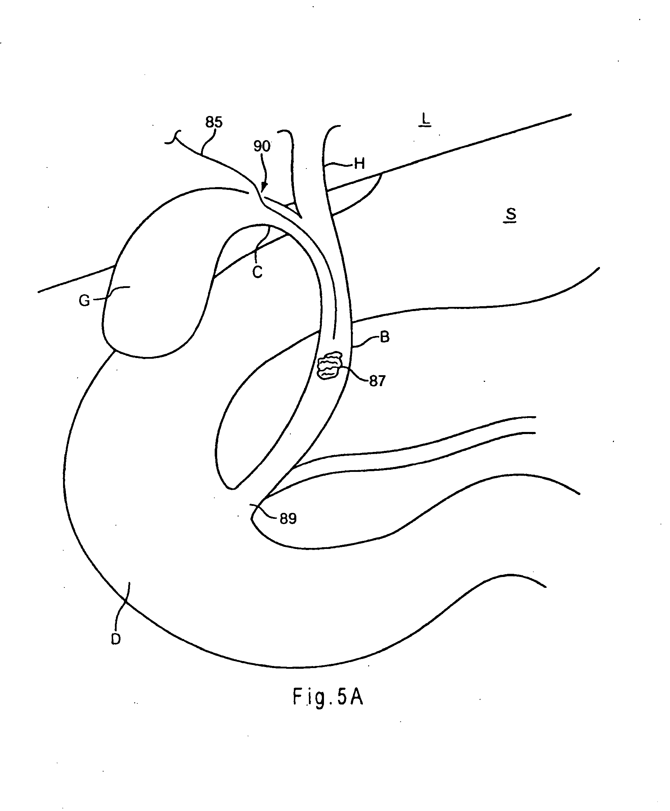

Referring now to FIGS. 5A-5E, a first exemplary method of using the claimed system for removing calculi, and in particular a gallstone, is described. As shown in FIG. 5A, the pertinent anatomy depicts cystic duct **C** leading from the gallbladder **G** into bile duct **B.** Hepatic duct **H** leads from liver **L** into bile duct **B.** The junction of cystic duct **C** and hepatic duct **H** form bile duct **B,** which extends towards sphincter of Oddi 89 and into duodenum **D.** Stomach S also empties into duodenum **D,** as shown in FIG. 5A. In this example, gallstone 87 has migrated from gallbladder **G** and has lodged within bile duct **B.** The gallstone may be trapped within bile duct B by the muscle of sphincter of Oddi 89.

As shown in FIGS. 5A-5E, bile duct **B** may be accessed laparoscopically using techniques that are known in the art. After an appropriate abdominal incision, small incision 90 is made in cystic duct **C.** Wire guide 85 then is inserted through incision 90 and fluoroscopically advanced through cystic duct **C** and into bile duct **B,** as shown in FIG. 5A.

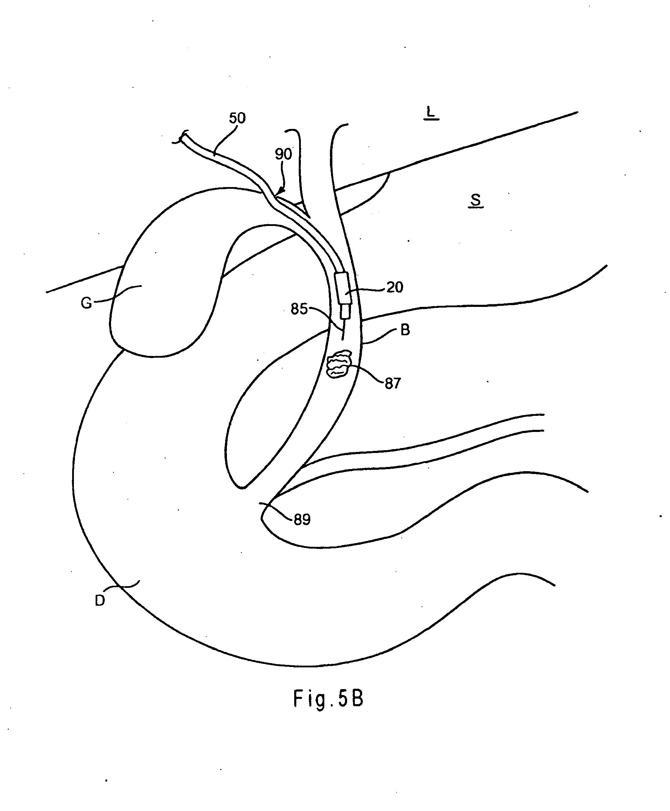

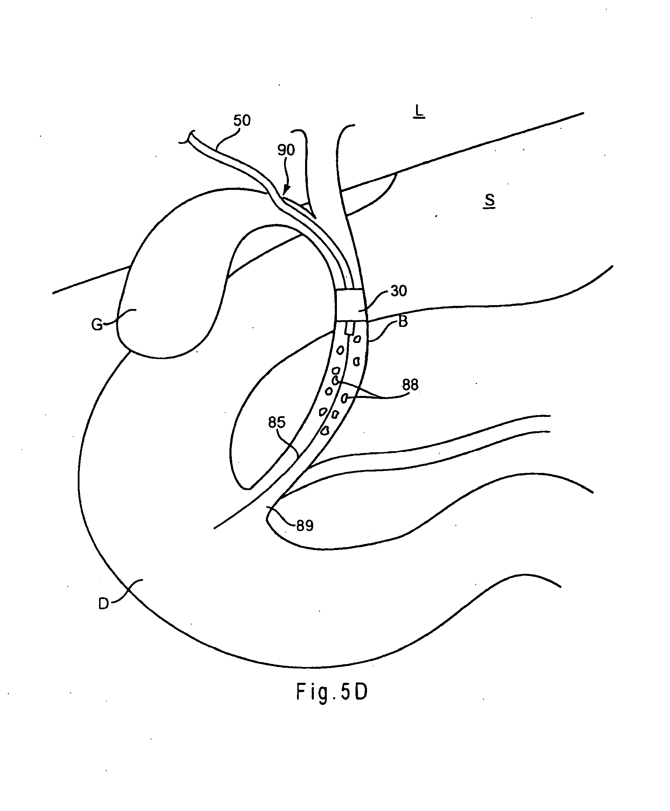

Referring now to FIG. 5B, in a next step cholangioscope 50 having balloon cuff 20 disposed thereon is advanced distally over wire guide 85 towards gallstone 87. Wire guide 85 preferably is disposed in auxiliary lumen 62 of cholangioscope 50 and guides the cholangioscope through cystic duct **C** and into bile duct **B.** At this time, optical elements 73 and 74 capture a close-up image of gallstone 87, which is disposed just distal to cholangioscope 50. If gallstone 87 is relatively large, as depicted, wire guide 85 is disposed just proximal (upstream) to the gallstone so that shock lithotripsy may be performed, as explained below with respect to FIG. 5C. If gallstone 87 is relatively small, such that lithotripsy is not needed, then wire guide 85 may be advanced distally (downstream) past gallstone 87 and through bile duct **B,** though sphincter of Oddi 89 and into duodenum **D,** and the method steps described below with respect to FIGS. 5D-5E may be performed.

In FIG. 5C, balloon 30 at the distal end of cholangioscope 50 is inflated to occlude a section of bile duct **B** proximal to gallstone 87. In a next step, shock wave lithotripsy probe 92 is inserted through proximal port 82 and working lumen 61 of cholangioscope 50 until probe 92 exits distal to the cholangioscope. At this time, optical elements 73 and 74 of FIG. 2 view the position of the distal end of probe 92 and further guide its movement towards gallstone 87. The physician then fine-tunes the positioning of probe 92 with respect to gallstone 87 in preparation for the intraductal shock wave lithotripsy procedure.

Shock waves may be generated, for example, using either electrohydraulic or laser technology. In an electrohydraulic lithotripsy procedure, a vaporizing fluid is delivered in the vicinity of gallstone 87 and voltage is applied to electrodes located at the distal end of probe 92 to produce shock waves at the surface of gallstone 87. If this technique is employed, the vaporizing fluid may be delivered through auxiliary lumen 62, with or without wire guide 85 disposed therein. Alternatively, the vaporizing fluid may be delivered through working lumen 61 via an annular space formed around an exterior surface of probe 92.

In a laser lithotripsy procedure, light is converted into thermal energy at the surface of gallstone 87. Various commercial electrohydraulic and laser lithotripsy systems are currently available for performing endoscopic lithotripsy.

The lithotripsy procedure disintegrates gallstone 87 to form multiple smaller stone fragments 88, as shown in FIG. 5D. Since balloon 30 is inflated, stone fragments 88 are situated distal (downstream) to cholangioscope 50 and are viewable by optical elements 73 and 74 of cholangioscope 50. At this time, the physician may determine whether additional lithotripsy is desirable on any of the stone fragments. If the stone fragments are small enough, then the physician may distally advance wire guide 85 through bile duct **B,** through sphincter of Oddi 89 and into duodenum **D,** as shown in FIG. 5D.

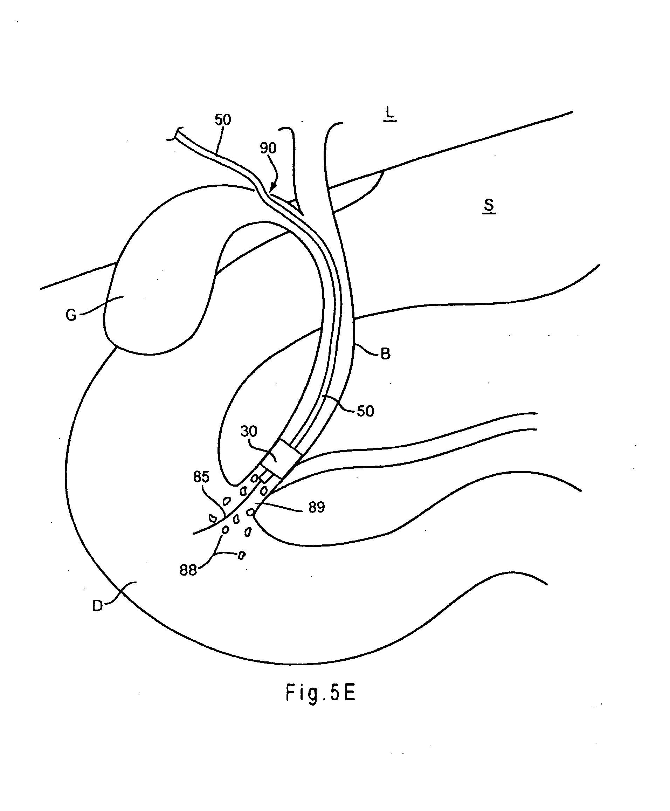

As shown in FIG. 5E, cholangioscope 50, with inflated balloon 30 securely disposed thereon, is then advanced distally (downstream) over wire guide 85 towards duodenum **D.** The distal advancement of cholangioscope 50 within bile duct **B** urges stone fragments 88 towards and through sphincter of Oddi 89. Balloon 30 may also be utilized to facilitate dilation of sphincter of Oddi 89 so as to permit stone fragments 88 to pass therethrough and into duodenum **D.** Once the stone fragments are in duodenum **D,** they will pass naturally through the patient via the intestinal pathway.

Once the stone fragments have been satisfactorily removed, balloon 30 is deflated, and cholangioscope 50 and wire guide 85 are removed from the patient's body. Incision 90 in cystic duct **C** may then be closed using techniques that are known in the art.

If necessary, balloon 30 may be inflated and deflated multiple times during the stone removal procedure using actuation device 46 of FIG. 2. For example, if a physician believes that stone fragments 85 remain in bile duct **B** after the method step described in FIG. 5E, then balloon 30 may be deflated, cholangioscope 50 may be retracted proximally (upstream) past the location of any suspected remaining stone fragments, balloon 30 may be re-inflated, and cholangioscope 50 may be advanced again distally to subsequently urge those fragments into duodenum **D.** If alternative cuff member 22' of FIG. 4 is employed, then cuff member 22' may be retracted and advanced independently of cholangioscope 50 to help urge stone fragments 85 into duodenum **D**.

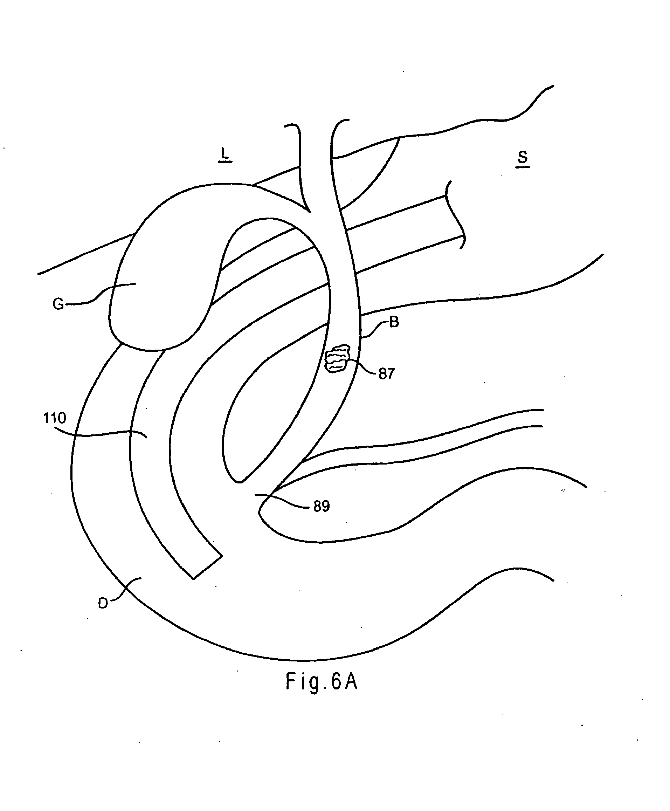

Referring now to FIGS. 6A-6F, an alternative exemplary method of using the claimed system for removing foreign matter, and in particular a gallstone, is described. Like in the method of FIGS. 5A-5E, gallstone 87 is located within bile duct **B**. In this embodiment, bile duct **B** may be accessed endoscopically using a mother-baby endoscope system. The system comprises a larger motherscope, i.e., a duodenoscope, and a smaller babyscope, i.e., a cholangioscope.

In FIGS. 6A-6F, balloon cuff 20 having cuff member 22 and ' balloon 30, as well as cholangioscope 50, preferably are provided in accordance with apparatus described above with respect to FIGS. 1-3. The apparatus may be used in conjunction with conventional duodenoscope 110 having proximal and distal ends and a working lumen (not shown) disposed therebetween. In a preferred embodiment, duodenoscope 110 is a side-viewing duodenoscope. In operation, duodenoscope 110 is inserted into a patient's mouth, through the esophagus, through stomach S, and into duodenum **D,** as schematically shown in FIG. 6A. The distal end of duodenoscope 110 is positioned in the vicinity of sphincter of Oddi 89.

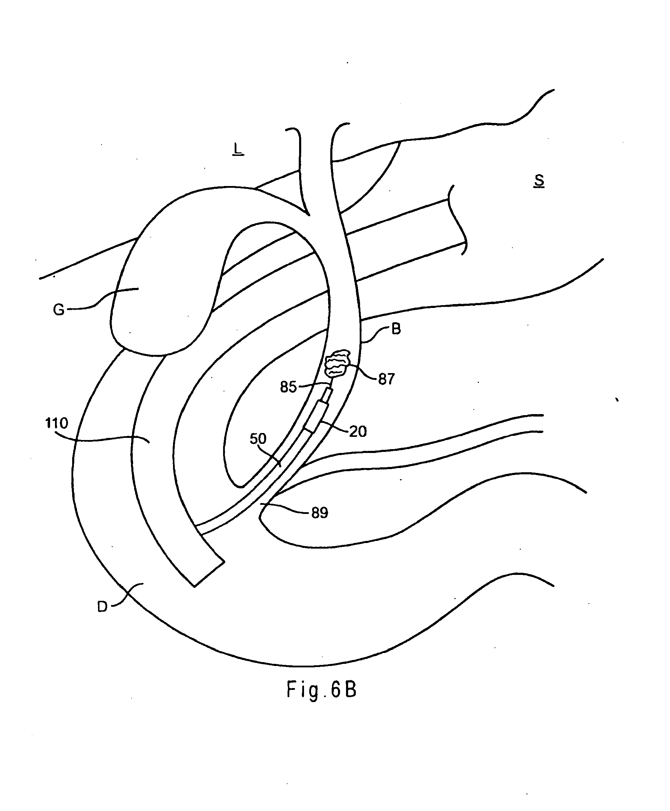

Referring now to FIG. 6B, wire guide 85 is inserted through the working lumen of duodenoscope 110. The distal end of wire guide 85 is advanced out of duodenoscope 110, through sphincter of Oddi 89, and into bile duct **B.** The distal end of cholangioscope 50 is then advanced over wire guide 85 and disposed proximal (downstream) of gallstone 87, as depicted in FIG. 6B. If necessary, a sphincterectomy may be performed at sphincter of Oddi 89 to facilitate access into bile duct **B** using techniques that are known in the art.

The working lumen of duodenoscope 110 may have an inner diameter of about 4.0 - 5.5 mm, while the overall diameter of duodenoscope 110 may be about 10-14 mm. Where the inner diameter of the working lumen of duodenoscope 110 is about 4.0 mm, cholangioscope 50 may comprise an outer diameter of about 3.4 mm and a working lumen 61 of about 1.2 to about 1.4 mm in diameter. Where the inner diameter of the working lumen of duodenoscope 110 is about 5.5 mm, cholangioscope 50 may comprise an outer diameter of about 4.0 mm and a working lumen 61 of about 1.7 mm in diameter.

Since balloon cuff 20 is disposed on an exterior surface of cholangioscope 50, cuff member 22 and balloon 30 generally add to the overall outer diameter of cholangioscope 50. Therefore, it is preferred that duodenoscope 110 has a relatively large working lumen, and/or cholangioscope 50 has a relatively small outer diameter, to facilitate passage of cholangioscope 50 and balloon cuff 20 through duodenoscope 110.

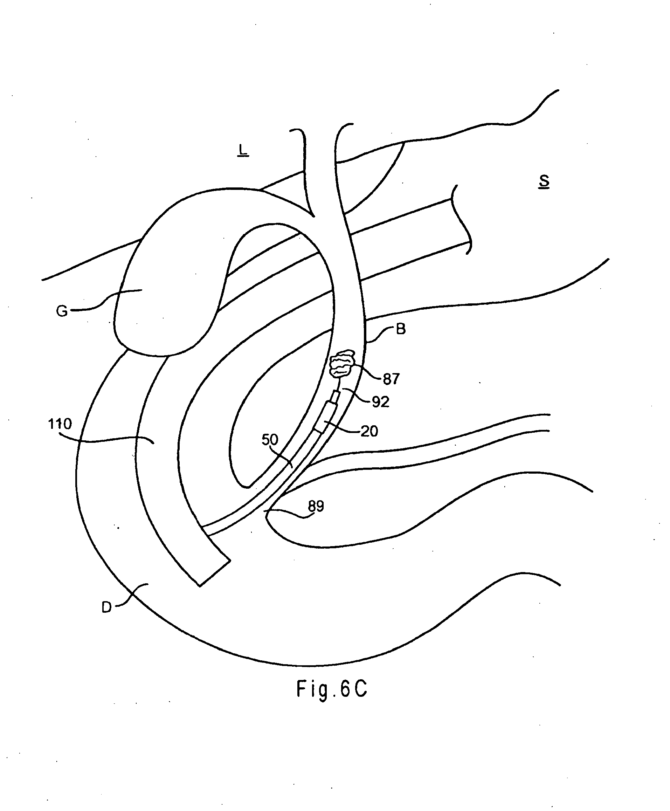

Referring now to FIG. 6C, in a next step, shock wave lithotripsy probe 92 is inserted through working lumen 61 of cholangioscope 50 until probe 92 exits distal to the cholangioscope. At this time, optical elements 73 and 74 view the position of the distal end of probe 92 and further guide its movement towards gallstone 87. The physician then fine-tunes the positioning of probe 92 with respect to gallstone 87 in preparation for the lithotripsy procedure. As mentioned above with respect to FIG. 5C, shock waves may be generated using either electrohydraulic or laser lithotripsy techniques.

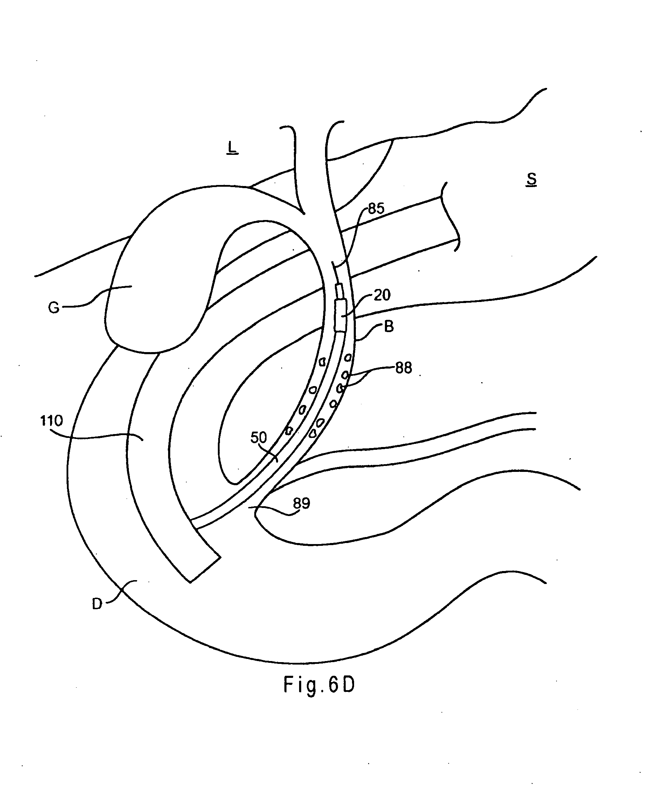

The lithotripsy procedure disintegrates gallstone 87 into smaller stone fragments 88, as shown in FIG. 6D. In a next step, probe 92 may be retracted proximally into working lumen 61 and wire guide 85 may be advanced distally (upstream) within bile duct **B**, as shown in FIG. 6D. Cholangioscope 50 is advanced over wire guide 85 with guidance from optical elements 73 and 74, or alternatively, using fluoroscopic guidance. Cholangioscope 50 is advanced distally until has passed some or all stone fragments 88.

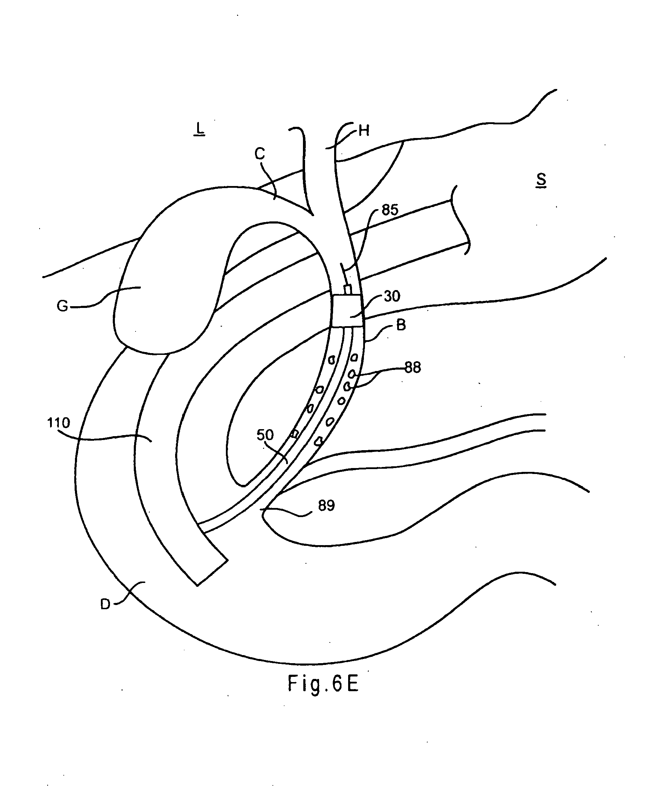

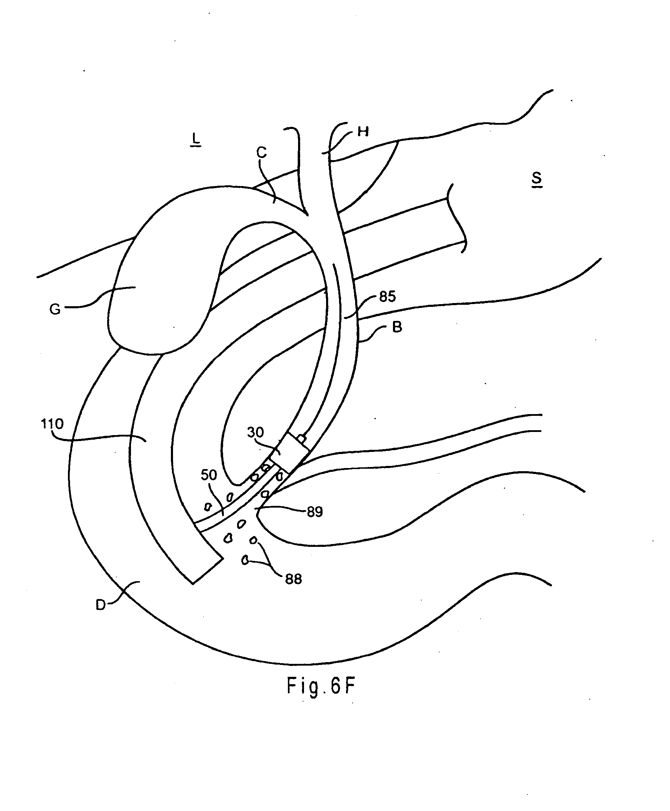

Referring now to FIGS. 6E-6F, in a next step balloon 30 is inflated via inflation means 40 and actuation device 46 (see FIG. 2). The balloon is inflated to engage an interior wall of bile duct **B** at a location distal to stone fragments 88, as shown in FIG. 6E. The physician may then retract cholangioscope 50 proximally to cause balloon 30 to urge stone fragments 88 towards sphincter of Oddi 89, as shown in FIG. 6F. Balloon 30 may also be utilized to facilitate dilation of sphincter of Oddi 89 so as to permit stone fragments 88 to pass more freely into duodenum **D.** Once the stone fragments are in duodenum **D,** they will pass naturally through the patient via the intestinal pathway.

It should be noted that if some stone fragments 88 are situated distal to the junction of cystic duct **C** and hepatic duct **H,** then a supplemental retrieval means, such as an extraction basket, may be employed if balloon 30 may not safely be advanced distally past the stone fragments. Further, if the initial shock wave lithotripsy procedure does not sufficiently disintegrate gallstone 87, then a supplemental shock wave lithotripsy procedure, or a mechanical lithotripsy procedure, may be performed.

It will be apparent that while intraductal shock wave lithotripsy has been described, other lithotripsy techniques may be used. For example, extracorporeal shock wave lithotripsy may be used to disintegrate a large stone, prior to the introduction of cholangioscope 50 and balloon cuff 20 into bile duct **B.** It will also be apparent that cholangioscope 50 and balloon cuff 20 may be used to remove smaller stone fragments without the need to perform a lithotripsy procedure. In such cases, cholangioscope 50 having balloon cuff 20 simply are inserted into bile duct **B**, balloon 30 is inflated, and the stone fragments are urged into duodenum **D,** as described above.

Advantageously, it is not required to use a separate balloon catheter or extraction basket disposed through the working channel of an endoscope to remove stone fragments from bile duct **B.** Therefore, there is no need to advance such components through the working lumen of the endoscope during the stone retrieval procedure. Further, as noted above, any endoscope may be retrofitted to employ balloon cuff 20, meaning that the balloon cuff may be added to an existing endoscope that does not currently employ such an external component suitable for performing the functions described above.

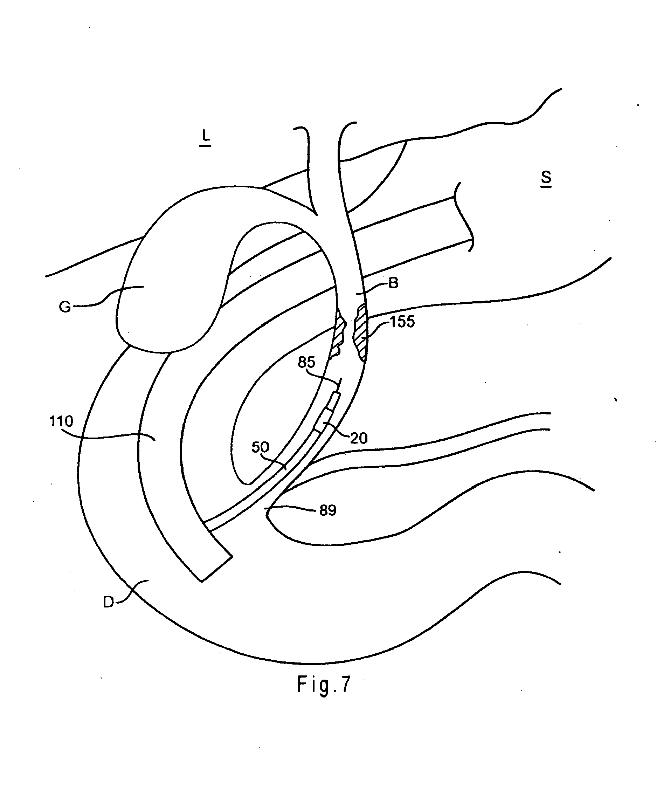

Finally, it will be apparent that while the above embodiments have described balloon cuff20 that may be used to treat gallstones that have migrated into the bile duct, the apparatus may be used to remove calculi or other particulate matter in other anatomical passages, such as kidney stones in the ureter or kidneys, and so forth. Alternatively, as depicted with respect to FIG. 7, balloon cuff 20 may be used to treat a stricture, such as biliary stricture 155. In the application depicted in FIG. 7, balloon 30 preferably comprises a substantially non-compliant material that may be used to dilate stricture 155. As will be apparent, wire guide 85 may be used to traverse stricture 155, and balloon 30 may be advanced over wire guide 85, aligned with stricture 155, and inflated to dilate the stricture.

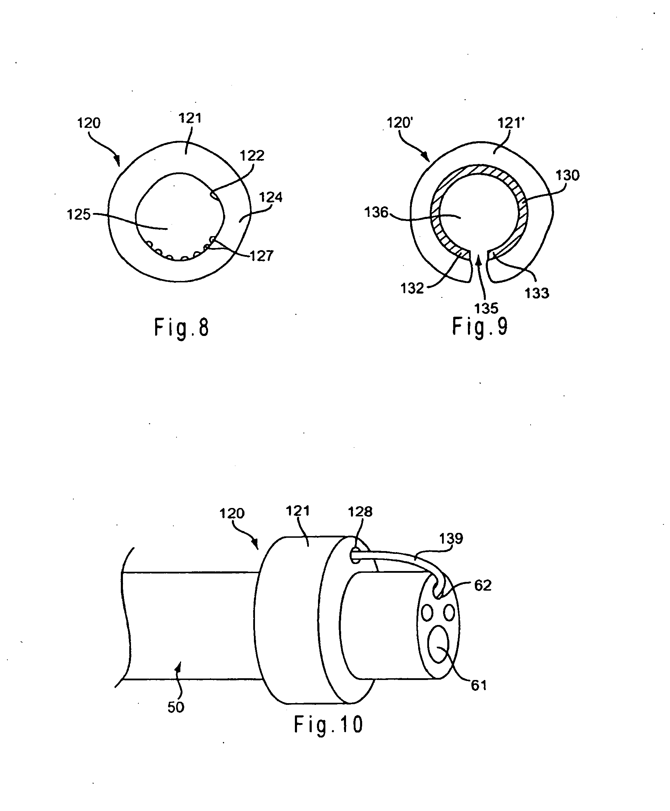

Referring now to FIGS. 8-10, alternative embodiments are described. In FIG. 8, cuff member 22 has been omitted and an alternative attachment structure is disclosed. Alternative balloon cuff 120 comprises generally donut-shaped or tubular-shaped balloon 121 having interior surface 122, exterior surface 124, and lumen 125 formed within interior surface 122. Balloon 121 may be elastically stretched in a radial direction in order to be disposed about the exterior surface ofcholangioscope 50. In this embodiment, the attachment structure is interior surface 122, i.e., the attachment structure is integral to the balloon. An engaging surface of interior surface 122 may comprise one or more frictional elements 127, or an adhesive material, to facilitate attachment of balloon 121 to the exterior surface of cholangioscope 50 and prevent slippage of the balloon.

In FIG. 9, balloon cuff 120' comprises alternative cuff member 130, which comprises a generally circular clip-shape having first end 132, second end 133, and lumen 136 formed therein. Cuff member 130 comprises gap 135 formed between ends 132 and 133, which allows some flexing of the ends to facilitate attachment around the exterior surface of cholangioscope 50. In this embodiment, balloon 121' is attached independently to first and second ends 132 and 133, as shown in FIG. 9. When inflated, balloon 121' may expand to fill in the space around gap 135, thereby forming a substantially complete tubular shape.

In an alternative embodiment, first and second ends 132 and 133 may comprise hook and loop fasteners (not shown), respectively, which are adapted to engage one another to secure balloon cuff 120' about an exterior surface of cholangioscope 50. In such embodiment, cuff member 130 may be flexible to permit partial overlapping of first and second ends 132 and 133. Other fastening means, such as Velcro®, may be employed to secure first and second ends 132 and 133 together.

Referring to FIG. 10, alternative balloon cuff 120 is shown disposed about an exterior surface of cholangioscope 50. Specifically, balloon 121 is elastically stretched to enlarge lumen 125, then passed over the distal end of cholangioscope 50, then relaxed to securely engage the exterior surface of cholangioscope 50. If balloon cuff 120' of FIG. 9 is employed, then cuff member 130 may be enlarged by moving ends 132 and 133 apart for placement over cholangioscope 50.

In either embodiment, the inflation means may comprise a small inflation tube 139 disposed between balloon 121 and the distal end of cholangioscope 50. Specifically, tube 139 may be permanently or removably attached to balloon 121 via port 128, then placed in fluid communication with lumen 62 at the distal end of cholangioscope 50. Lumen 62 then serves as a dedicated inflation lumen. An adhesive or other means may be used to secure tube 139 to lumen 62. Advantageously, using this technique, the inflation means need not extend externally along the entire length of cholangioscope 50.

While various embodiments of the invention have been described, it will be apparent to those of ordinary skill in the art that many more embodiments and implementations are possible within the scope of the invention. Accordingly, the invention is not to be restricted except in light of the attached claims and their equivalents.

## Claims

1. A system for removing a stone in a body passage, the system comprising:
a first endoscope (50) having proximal and distal ends, an exterior surface, and a working lumen disposed between the proximal and distal ends;
a balloon (30) having deflated and inflated states; and
inflation means (40) in fluid communication with the balloon,
wherein the balloon is configured to urge stone fragments in a direction along the body passage when in the inflated state,
**characterised in that** the system further comprises an attachment structure (22) disposed around the exterior surface of the first endoscope; and **in that** the balloon is coupled to the attachment structure.

2. The system of claim 1 wherein the attachment structure comprises an interior surface of the balloon which is tubular shaped.

3. The system of claim 1 wherein the attachment structure comprises a cuff member (22) having proximal and distal ends and a lumen disposed therebetween.

4. The system of claim 3 wherein the balloon is coupled to an exterior surface of the cuff member.

5. The system of claim 1 further comprising a second endoscope having proximal and distal ends and a working lumen disposed therebetween, wherein the first endoscope having the attachment structure disposed thereon is adapted for insertion through the working lumen of the second endoscope.

6. The system of claim 1 wherein the balloon comprises a material having a softness and elasticity that facilitates longitudinal movement of the balloon along the body passage when the balloon is-in the inflated state and engaged with a wall of the body passage.

7. The system of claim 1 wherein the balloon comprises a lubricious coating that facilitates longitudinal movement of the balloon along the body passage when the balloon is in the inflated state and engaged with a wall of the body passage.

8. The system of claim 1 wherein the balloon comprises a material having a low coefficient of friction that facilitates longitudinal movement of the balloon along the body passage when the balloon is in the inflated state and engaged with a wall of the body passage.

9. The system of claim 2 wherein the interior surface of the balloon comprises an engaging surface comprising a frictional element or an adhesive to facilitate attachment to the exterior surface of the first endoscope.

10. The system of claim 3 wherein the cuff member comprises an elastic member, wherein the lumen has a first inner diameter in a relaxed state and a larger second diameter in an expanded state, wherein the cuff member is adapted to be placed over the first endoscope in the expanded state.

11. The system of claim 10 wherein the first inner diameter of the cuff member in the relaxed state is smaller than an outer diameter of the first endoscope to permit the cuff member to be elastically secured about the exterior surface of the first endoscope.

12. The system of claim 1 wherein the balloon comprises an opening disposed therethrough, wherein the opening is adapted to be disposed around an exterior surface of the first endoscope.

13. The system of claim 12 wherein the balloon comprises a donut-shape.

14. The system of claim 1 wherein the working lumen of the first endoscope is sized to permit advancement of an intraductal shock wave lithotripsy device.

## Patentansprüche

1. System zur Entfernung eines Steins aus einem Körpergang, wobei das System Folgendes umfasst:
ein erstes Endoskop (50) mit proximalen und distalen Enden, einer Außenseite und einem zwischen den proximalen und distalen Enden angeordneten Arbeitslumen;
einen Ballon (30) mit entleerten und aufgeblasenen Zuständen sowie ein Aufblasmittel (40), das mit dem Ballon in Fluidverbindung steht,
worin der Ballon so konfiguriert ist, dass er im aufgeblasenen Zustand Steinfragmente in einer Richtung entlang des Körpergangs drückt,
**dadurch gekennzeichnet, dass** das System ferner eine Befestigungskonstruktion (22) umfasst, die um die Außenseite des ersten Endoskops angeordnet ist, und dass der Ballon mit der Befestigungskonstruktion verbunden ist.

2. System nach Anspruch 1, worin die Befestigungskonstruktion eine röhrenförmige Innenseite des Ballons umfasst.

3. System nach Anspruch 1, worin die Befestigungskonstruktion ein Manschettenelement (22) mit proximalen und distalen Enden und einem dazwischen angeordneten Lumen umfasst.

4. System nach Anspruch 3, worin der Ballon mit einer Außenseite des Manschettenelements verbunden ist.

5. System nach Anspruch 1, das ferner ein zweites Endoskop mit proximalen und distalen Enden und einem dazwischen angeordneten Arbeitslumen umfasst, worin das erste Endoskop mit der darauf angeordneten Befestigungskonstruktion durch das Arbeitslumen des zweiten Endoskops eingeführt werden kann.

6. System nach Anspruch 1, worin der Ballon ein Material umfasst, das so weich und elastisch ist, dass eine Längsbewegung des Ballons entlang dem Körpergang erleichtert wird, wenn sich der Ballon in seinem aufgeblasenen Zustand befindet und mit einer Wand des Körpergangs in Eingriff steht.

7. System nach Anspruch 1, worin der Ballon eine Gleitbeschichtung umfasst, die eine Längsbewegung des Ballons entlang dem Körpergang erleichtert, wenn sich der Ballon in seinem aufgeblasenen Zustand befindet und mit einer Wand des Körpergangs in Eingriff steht.

8. System nach Anspruch 1, worin der Ballon ein Material mit einem niedrigen Reibungskoeffizienten umfasst, das eine Längsbewegung des Ballons entlang dem Körpergang erleichtert, wenn sich der Ballon in seinem aufgeblasenen Zustand befindet und mit einer Wand des Körpergangs in Eingriff steht.

9. System nach Anspruch 2, worin die Innenseite des Ballons eine Eingriffsfläche mit einem Reibungselement oder einem Klebstoff zur Erleichterung der Befestigung an der Außenseite des ersten Endoskops umfasst.

10. System nach Anspruch 3, worin das Manschettenelement ein elastisches Element umfasst, worin das Lumen einen ersten Innendurchmesser in einem entspannten Zustand und einen größeren zweiten Durchmesser in einem aufgeweiteten Zustand aufweist, worin das Manschettenelement im aufgeweiteten Zustand über dem ersten Endoskop platziert werden kann.

11. System nach Anspruch 10, worin der erste Innendurchmesser des Manschettenelements im entspannten Zustand kleiner ist als ein Außendurchmesser des ersten Endoskops, damit das Manschettenelement elastisch um die Außenseite des ersten Endoskops fixiert werden kann.

12. System nach Anspruch 1, worin der Ballon eine Öffnung durch ihn hindurch umfasst, worin die Öffnung um eine Außenseite des ersten Endoskops angeordnet werden kann.

13. System nach Anspruch 12, worin der Ballon donutförmig ist.

14. System nach Anspruch 1, worin das Arbeitslumen des ersten Endoskops eine solche Größe aufweist, dass eine intraduktale Stoßwellenlithotripsievorrichtung durchgeschoben werden kann.

## Revendications

1. Système pour retirer un calcul dans un canal anatomique, le système comprenant :
un premier endoscope (50) comportant des extrémités proximale et distale, une surface extérieure et une lumière de travail disposée entre les extrémités proximale et distale ;
un ballon (30) présentant des états dégonflé et gonflé ; et
un moyen de gonflage (40) en communication fluide avec le ballon,
dans lequel le ballon est configuré pour pousser, quand il est gonflé, des fragments de calcul dans une direction donnée le long du canal anatomique, **caractérisé en ce que** le système comprend en outre une structure de fixation (22) disposée autour de la surface extérieure du premier endoscope et **en ce que** le ballon est associé à la structure de fixation.

2. Système selon la revendication 1, dans lequel la structure de fixation comprend la surface intérieure du ballon, qui est de forme tubulaire.

3. Système selon la revendication 1, dans lequel la structure de fixation comprend un élément de manchette (22) comportant des extrémités proximale et distale et une lumière disposée entre elles.

4. Système selon la revendication 3, dans lequel le ballon est associé à la surface extérieure de l'élément de manchette.

5. Système selon la revendication 1, comprenant en outre un second endoscope comportant des extrémités proximale et distale et une lumière de travail disposée entre elles, dans lequel le premier endoscope comportant la structure de fixation disposée sur lui est apte à être introduit par la lumière de travail du second endoscope.

6. Système selon la revendication 1, dans lequel le ballon comprend une matière présentant une douceur et une élasticité qui facilitent le mouvement longitudinal du ballon le long du canal anatomique quand le ballon est en état gonflé et au contact de la paroi du canal anatomique.

7. Système selon la revendication 1, dans lequel le ballon comprend un revêtement lubrifiant qui facilite le mouvement longitudinal du ballon le long du canal anatomique quand le ballon est en état gonflé et au contact de la paroi du canal anatomique.

8. Système selon la revendication 1, dans lequel le ballon comprend une matière ayant un faible coefficient de frottement qui facilite le mouvement longitudinal du ballon le long du canal anatomique quand le ballon est en état gonflé et au contact de la paroi du canal anatomique.

9. Système selon la revendication 2, dans lequel la surface intérieure du ballon comprend une surface d'engagement comprenant un élément de friction ou un adhésif pour faciliter sa fixation à la surface extérieure du premier endoscope.

10. Système selon la revendication 3, dans lequel l'élément de manchette comprend un élément élastique, dans lequel la lumière a un premier diamètre intérieur dans une situation relâchée et un second diamètre intérieur plus grand dans une situation dilatée, dans lequel l'élément de manchette est apte à être placé, quand il est dilaté, par-dessus le premier endoscope.

11. Système selon la revendication 10, dans lequel le premier diamètre intérieur de l'élément de manchette en situation relâchée est inférieur au diamètre extérieur du premier endoscope afin de permettre à l'élément de manchette d'être fixé élastiquement autour de la surface extérieure du premier endoscope.

12. Système selon la revendication 1, dans lequel le ballon comprend une ouverture qui le traverse de part en part, dans lequel l'ouverture est apte à être disposée autour de la surface extérieure du premier endoscope.

13. Système selon la revendication 12, dans lequel le ballon présente une forme de beignet.

14. Système selon la revendication 1, dans lequel la lumière de travail du premier endoscope a une dimension permettant la progression d'un dispositif intracanalaire de lithotritie par ondes de choc.
